# EUROPEAN PATENT SPECIFICATION

(11) **EP 1 778 077 B1**
(45) Date of publication and mention of the grant of the patent: **14.01.2015**
(21) Application number: 05826810.3
(22) Date of filing: 25.07.2005
(51) Int. Cl.: A61B 19/00

(54) **WIRELESS MARKERS FOR ANCHORING WITHIN A HUMAN BODY**
DRAHTLOSE VERANKERUNGSMARKER FÜR EINEN MENSCHLICHEN KÖRPER
MARQUEURS SANS FIL POUR FIXATION DANS LE CORPS HUMAIN

(30) Priority: 23.07.2004 US 590452 P
(43) Date of publication of application: 02.05.2007
(73) Proprietor: Varian Medical Systems, Inc., Palo Alto, CA 94304-1038 (US)
(72) Inventor: SEILER, Keith, Issaquah, WA 98027 (US); HADFORD, Eric, Snohomish, WA 98296 (US); PURDY, Lynn M., Sammamish, WA 98075 (US)
(74) Representative: Hoarton, Lloyd Douglas Charles
(86) International application number: PCT/US2005/026430
(87) International publication number: WO 2006/020377

(56) References cited:
- WO-A1-02/39917
- WO-A1-02/39917
- WO-A1-96/03925
- WO-A2-00/16686
- WO-A2-2004/061460
- WO-A2-2005/084572
- US-A1- 2002 120 200
- US-A1- 2003 052 785
- US-A1- 2003 181 794
- US-A1- 2003 181 794
- US-A1- 2004 124 105
- US-B1- 6 385 482
- US-B2- 6 746 404
- US-B2- 6 752 154

## Description

### TECHNICAL FIELD

The present invention is directed toward implantable markers with signal transmitters that wirelessly transmit location signals from within a patient's body. In particular, several aspects of the invention relate to anchoring or fastening markers to their surrounding medium to prevent them from changing location.

### BACKGROUND

Many medical procedures require monitoring or treating an internal tissue mass or other parts within a human body. In such applications, medical procedures must accurately locate a small target location within a soft tissue region, an organ, a bone structure, or another body part (*e.g*., colon, vascular system, etc.). The small target location can be a lesion, polyp, tumor, or another area of interest for monitoring or treatment. For example, it is particularly important to know or estimate the precise location of the target in radiation oncology because it is desirable to accurately determine the accumulated dosage applied to the target and it is detrimental to expose adjacent body parts to the radiation. In applications for treating prostate cancer, for example, it is detrimental to irradiate the colon, bladder or other neighboring body parts with the high-intensity radiation beam. Surgical applications, such as breast surgery and other procedures involving soft tissue, also require knowing the precise location of a target because a lesion in soft tissue is not necessarily fixed relative to external landmarks on the patient.

Some applications are particularly challenging because physicians often need to treat small, non-palpable lesions that cannot be observed. This problem is compounded in soft tissue applications because the soft tissue is mobile and can move with respect to a reference point on the patient. In the case of breast cancer, for example, the location of a non-palpable lesion in the breast is identified at a pre-operative stage using an imaging system. The surgical procedure or radiation treatment, however, occurs at a subsequent point in time, and the patient, and consequently the tissue and the lesion, are typically in a different position during such processes compared to the pre-operative imaging stage. The physician, therefore, generally estimates the location of lesion during the process.

One problem with treating non-palpable lesions in soft tissues is that the physicians may incorrectly estimate the location of the target. As a result, the physician may not remove the entire lesion or cause undesirable collateral damage to healthy tissue by removing a significant amount of tissue proximate to the lesion. The same kind of problems may occur in case of radiation. In general, during the radiation or surgical procedure it is desirable, and in many cases it is vital, to know the precise location of the targets.

In medical fields, to accurately target portions of a human body, various devices are used. For example, different imaging systems have been used to locate areas or particular targets in a patient before performing radiation oncology or surgical procedures. In many medical applications, however, imaging techniques by themselves are not well suited for accurately identifying the actual location of a target. And although x-ray, Magnetic Resonance Imaging (MRI), CT and other imaging techniques are useful to locate targets within the body at a pre-operative stage of a procedure, they are often not suitable or difficult to use in real time during surgery or radiation therapy.

Another technique to locate a target in a patient is to implant a marker relative to the target. For example, implantable markers that generate a signal have been proposed for use to locate a selected target in a patient in radiation oncology procedures. U.S. Patent No. 6,385,482 B1 issued to Boksberger et al. discloses a device having an implanted emitter unit located inside or as close as possible to a target object, and a plurality of receiver units that are located outside of the patient. The wired device disclosed in Boksberger, however, may not be suitable for use in radiation oncology and many surgical procedures because it is impractical to leave a wired marker implanted in a patient for the period of time of such procedures (e.g., five to forty days).

Another example is the U.S. Patent No. 5,397,329 to Allen, which describes fiducial implants for a human body that can be detected in x-rays. The fiducial implants are implanted into the skull or other bone structure beneath the skin. The fiducial implants in Allen are also spaced sufficiently far apart from one another to define a plane that can be identified by the imaging system and is used in connection with creation of images of a body portion of interest. The systems disclosed in Allen generally function effectively only when the devices defining the body portion of interest are fixed structures, such as bone, and thus they are not well suited to operate as intended when the devices are inserted in amorphous, pliable tissue.

Another recent method for locating a target within the body includes a wireless implantable marker configured to be implanted, surgically or percutaneously, into a human body relative to a target location. The markers include a casing and a signal element in the casing that wirelessly transmits location signals in response to an excitation energy. One concern of using implanted markers in soft tissues is that the markers may move within the patient after implantation. To resolve this concern, Calypso Medical Technologies, Inc. previously developed several anchors and fasteners for securing the markers to soft tissue structures, as disclosed in U.S. Application No. 10/438,550. Although these anchors work well for percutaneous implantation, they may be improved for surgical applications. Therefore, it would be desirable to further develop markers for surgical and/or percutaneous implantation.

WO 02/39917 A1, WO 00/16686 A2, US 2003/05 2 785 and

US-A-2003/0181794 disclose a sensor unit which may be implanted and secured within a human body.

The present invention seeks to provide an improved wireless marker.

According to one aspect of the present invention, there is provided a wireless marker as defined in claim 1 hereinafter.

### BRIEF DESCRIPTION OF THE DRAWINGS

Figure 1 is an isometric view of a marker.
Figure 2 is an isometric view of a marker in accordance with an embodiment of the invention.

### DETAILED DESCRIPTION

### A. Overview

The following disclosure describes several wireless markers configured to be implanted and anchored within a human in a manner that prevents the markers from migrating from the implantation site. The markers are highly suitable for use in surgical radiation therapy and other applications to determine the location and orientation of a target of the patient. The markers can be anchored or fastened to tissue or another anatomical medium in a number of invasive and non-invasive ways for surgical or percutaneous implantation.

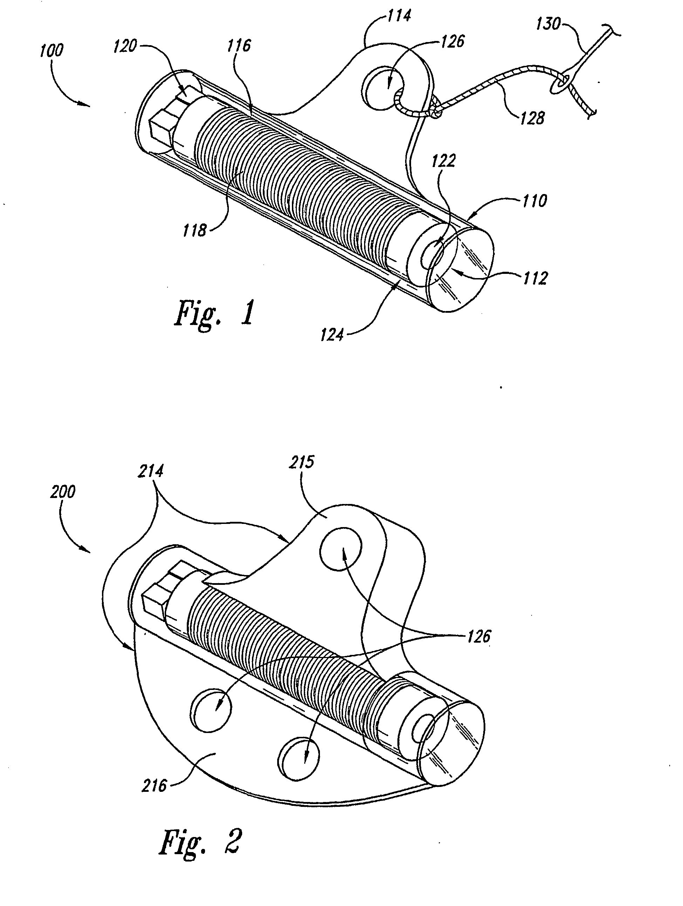

Several embodiments and features of markers with anchors in accordance with claimed and unclaimed embodiments of the invention are set forth and described in Figures 1-2. In other embodiments, the markers can include additional or different features than those shown in Figures 1-2. Additionally, several embodiments of markers may not include all the features shown in these Figures. For the purposes of brevity, like reference numbers refer to similar or identical components of the markers in Figures 1-2.

One embodiment of a marker for localizing a target of a patient comprises a casing, a magnetic transponder at least partially received in the casing, and an anchor carried by the casing. The casing is a biocompatible barrier configured to be implanted in the patient. The casing can be a generally cylindrical capsule that is sized to fit within a needle for percutaneous implantation, but the casing can have other geometric shapes, sizes, and configurations in other applications. For example, the casing can be larger for surgical applications. The magnetic transponder produces a wirelessly transmitted magnetic field in response to a wirelessly transmitted excitation energy. The magnetic transponder can further comprise a magnetic core, a coil wrapped around the core, and a capacitor coupled to the coil. The anchor, which can project from the casing, secures the marker to an anatomical structure to prevent the marker from moving from the implantation site. The anchor may be sutured to the anatomical structure or attached to the anatomical structure by mechanical members or chemical attributes.

The anchor may protrude from the casing and have a hole through which a needle, suture line, or other suture material can pass. The hole defines a suture retainer in the protrusion. The anchor may be an integral extension of the casing, or the anchor can be a separate extension embedded in or attached to the casing. The embedded part of an anchor may be formed such that it creates a strong footing in the casing material to better support the protruding portion of the anchor. The anchor, for example, can be a fin or flange with one or more holes for receiving the suture material. A plurality of anchors may protrude from a casing.

An anchorable marker for localizing a target of a patient comprises a casing, a transponder that produces a wirelessly transmitted magnetic field in response to a wirelessly transmitted excitation field, and an anchor partially embedded within the casing. The anchor can have a shape and/or material that pierces, engages or otherwise interfaces with the anatomical anchoring site such that the marker cannot be easily dislodged. Such embodiments are also well suited for surgical or percutaneous implantation. The anchor can have suturing material, such as a needle and suture wire pre-attached to the anchor. For example, the needle can be threaded through the anchor and the suture line can be pre-tied to the anchor so that the surgeon can immediately suture the marker to the tissue upon opening of the package. Other combinations of a suturable marker and suturing material are possible.

In the following description, several specific details are presented to provide a thorough understanding of the embodiments of the invention. One skilled in the relevant art, however, will recognize that the invention can be practiced without one or more of the specific details, or the invention can be practiced in combination with or without other components. Furthermore, the particular features, structures, implementation, or characteristics may be combined in any suitable manner in one or more embodiments. In other instances, well-known implementations or operations are not shown or described in detail to avoid obscuring aspects of various embodiments of the invention.

### B. Embodiments of Anchorable Markers

Figures 1-2 are isometric views of markers 100-200. Referring to Figure 1, a marker 100 includes a casing 110, a magnetic transponder 112 (e.g., a resonating circuit) at least partially encased in the casing 110, and an anchor 114. The casing 110 is a biocompatible barrier, which can be made from plastics, ceramics, glass or other suitable materials, and the casing 110 is configured to be implanted in the patient. The casing 110 can be a generally cylindrical capsule that is sized to fit within a needle for percutaneous implantation. For example, the casing 110 can have a diameter of approximately 2 mm or less. In surgical applications, the casing can have a larger diameter and other configurations.

The magnetic transponder 112 can include a resonating circuit that produces a wirelessly transmitted signal in response to a wirelessly transmitted excitation field. In one embodiment, the magnetic transponder 112 comprises a coil 116 defined by a plurality of windings of a conductor 118. Many embodiments of the magnetic transponder 112 also include a capacitor 120 coupled to the coil 116. The coil 116 can resonate at a resonant frequency solely using the parasitic capacitance of the windings without having a capacitor, or the resonant frequency can be produced using the combination of the coil 116 and the capacitor 120. The coil 116 accordingly defines a signal transmitter that generates an alternating magnetic field at the selected resonant frequency in response to the excitation energy either by itself or in combination with the capacitor 120. The coil 116 generally has 800-2000 turns, and the windings are preferably wound in a tightly layered coil.

The magnetic transponder 112 can further include a core 122 composed of a material having a suitable magnetic permeability. For example, the core 122 can be a ferromagnetic element composed of ferrite or another material. Suitable embodiments of magnetic transponders are disclosed in U.S. Patent Application Nos. 10/334,698 and 10/746,888. The magnetic transponder 112 can be secured to the casing 110 by an adhesive 124.

The anchor 114 protrudes from the marker casing 110. The anchor 114 can be an integral extension of the casing 110, or the anchor 114 can be a separate component attached to and/or embedded in the casing 110. When the anchor 114 is a separate component, it can be made from a suitable biocompatible material, such as metals, metal alloys, or polymers and other synthetic materials. An example of one such material is spring steel, although other "memory" metal alloys may be suitable. The anchor 114 shown In Figure 1 is a fin or flange having a suture retaining element 126 configured to hold a suture line. In the case of a flange, the anchor 114 can be as thick as the diameter of the casing 110. The suture retainer 126 is an enclosed hole enclosed. A suturing material, such as suture line 128 and needle 130, is pre-attached to the anchor 114 in the marker shown in Figure 1.

Figure 2 is an isometric view of a marker 200 in accordance with an embodiment of the invention. The marker 200 differs from the marker 100 in that the marker 200 includes an anchor 214 having a first anchor member 215, a second anchor member 216, and a plurality of suture retainers 126. The first anchor member 215 can be a fin or flange similar to the anchor 114 described above. The second anchor member 216 can be a fin or flange extending along a substantial portion of the length of the marker, and the second anchor member 216 has a plurality of suture retainers 126. This embodiment allows a surgeon to secure the marker from different directions to multiple points, and it provides more surface contact area to restrict movement of the marker.

From the foregoing, it will be appreciated that specific embodiments of the invention have been described herein for purposes of illustration, but that various modifications may be made. For example, the anchors can be composed of more than one material, or the anchors of the various embodiments can be interchanged or combined with each other. Additionally, some surgically implantable markers are also well adapted for percutaneous implantation. In cases of percutaneous implantation the anchors will lodge in the surrounding tissue instead of being sutured to the tissue. In some cases, depending on the geometry of the anchor, the surrounding tissue may grow into holes or other interstitial spaces of the anchor. The cylindrically shaped markers with anchors only at one end, are suited for percutaneous as well as surgical implantation. Furthermore, the anchors can further comprise drug eluting surfaces that contain drugs to promote tissue growth, provide antibodies, etc. Anchoring capacity can also be enhanced by adding adhesive material to the anchor and/or the casing. Accordingly, the invention is not limited except as by the claims.

## Claims

1. A wireless marker for implantation into a human body, comprising:
a casing;
a signal element at least partially encased in the casing, the signal element being configured to wirelessly transmit a location signal in response to a wirelessly transmitted excitation energy; and
a first anchor flange projecting from one portion of the casing.
a second anchor flange projecting from another portion of the casing, **characterised in that** the second anchor flange extends along a substantial portion of the length of
the marker, the first anchor flange has a suture retainer which comprises a hole through the first anchor flange and the second anchor flange has a plurality of suture retainers which comprise a plurality of holes through the second anchor flange, each suture retainer being configured to receive a suture line.

2. The wireless marker of claim 1 wherein the signal element comprises a magnetic permeable core, a coil wrapped around the core, and a capacitor electrically connected to the coil.

3. The wireless marker of any preceding claim, wherein a suture line is attached to at least one of the suture retainers.

4. The wireless marker of any preceding claim, wherein at least one of the anchors further comprises adhesive material that is configured to bind and/or adhere to tissue.

5. The wireless marker of claim 1 wherein at least one of the anchors further comprises a drug.

6. The wireless marker of claim 1 wherein the first anchor flange is as thick as the casing.

## Patentansprüche

1. Drahtloser Marker zur Implantation in einen menschlichen Körper,
umfassend:
ein Gehäuse;
ein Signalelement, das wenigstens teilweise im Gehäuse eingeschlossen ist, wobei das Signalelement konfiguriert ist, ein Lokalisierungssignal, als Reaktion auf eine drahtlos gesendete Anregungsenergie, drahtlos zu senden; und
einen ersten Verankerungsflansch, der aus einem Teil des Gehäuses hervorsteht,
einen zweiten Verankerungsflansch, der aus einem weiteren Teil des Gehäuses hervorsteht, **dadurch gekennzeichnet, dass** sich der zweite Verankerungsflansch entlang eines wesentlichen Teils der Länge des Markers erstreckt, wobei der erste Verankerungsflansch einen Nahtmaterialhalter aufweist, der ein Loch durch den ersten Verankerungsflansch umfasst und der zweite Verankerungsflansch eine Vielzahl von Nahtmaterialhaltern aufweist, die eine Vielzahl von Löchern durch den zweiten Verankerungsflansch aufweisen, wobei jeder Nahtmaterialhalter konfiguriert ist, einen Nahtfaden zu empfangen.

2. Drahtloser Marker nach Anspruch 1, wobei das Signalelement einen magnetischen durchlässigen Kern, eine um den Kern gewickelte Spirale und einen an die Spirale elektrisch angeschlossenen Kondensator umfasst.

3. Drahtloser Marker nach einem beliebigen vorangehenden Anspruch, wobei ein Nahtfaden wenigstens an einem der Nahtmaterialalter befestigt ist.

4. Drahtloser Marker nach einem beliebigen vorangehenden Anspruch, wobei wenigstens eine der Verankerungen ferner Klebematerial umfasst, das konfiguriert ist, sich an Gewebe zu binden und/oder zu haften.

5. Drahtloser Marker nach Anspruch 1, wobei wenigstens eine der Verankerungen ferner eine Droge umfasst.

6. Drahtloser Marker nach Anspruch 1, wobei der erste Verankerungsflansch so dick wie das Gehäuse ist.

## Revendications

1. Marqueur sans fil destiné à être implanté dans un corps humain, comprenant :
une enveloppe ;
un élément de signal au moins partiellement enveloppé dans l'enveloppe, l'élément de signal étant configuré pour transmettre sans fil un signal de localisation en réponse à une énergie d'excitation transmise sans fil ; et
un premier rebord d'ancrage se projetant à partir d'une partie de l'enveloppe ;
un second rebord d'ancrage se projetant à partir d'une autre partie de l'enveloppe, **caractérisé en ce que** le second rebord d'ancrage s'étend suivant une partie considérable de la longueur du marqueur, le premier rebord d'ancrage ayant un système de retenue de suture comprenant un orifice traversant le premier rebord d'ancrage, et le second rebord d'ancrage ayant une pluralité de systèmes de retenue de suture comprenant une pluralité d'orifices traversant le second rebord d'ancrage, chaque système de retenue de suture étant configuré pour recevoir une ligne de suture.

2. Marqueur sans fil selon la revendication 1, dans lequel l'élément de signal comprend un noyau perméable magnétique, une bobine enroulée autour du noyau, et un condensateur connecté électriquement à la bobine.

3. Marqueur selon l'une quelconque des revendications précédentes, dans lequel une ligne de suture est attachée à au moins un des systèmes de retenue de suture.

4. Marqueur selon l'une quelconque des revendications précédentes, dans lequel au moins un des ancrages comprend en outre un matériau adhésif conçu pour se lier et/ou adhérer à un tissu.

5. Marqueur sans fil selon la revendication 1, dans lequel au moins un des ancrages comprend un médicament.

6. Marqueur sans fil selon la revendication 1, dans lequel le premier rebord d'ancrage est aussi épais que l'enveloppe.
